(19)

Europäisches
Patentamt

European
Patent Office

Office européen
des brevets

(11) **EP 1 783 647 A2**

(12) **EUROPEAN PATENT APPLICATION**

(43) Date of publication:
**09.05.2007 Bulletin 2007/19**

(51) Int Cl.:
***G06F 19/00*** (2006.01)       ***G06F 17/30*** (2006.01)

(21) Application number: **06121967.1**

(22) Date of filing: **09.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **10.10.2005 FI 20055548**

(71) Applicant: **Medicel Oy
00290 Helsinki (FI)**

(72) Inventors:
- **Volanen, Jussi
  Espoo 02230 (FI)**
- **Helttunen, Juha
  Mäntsälä 04600 (FI)**
- **Leppänen, Tero
  Espoo 02650 (FI)**

(74) Representative: **Virkkala, Jukka Antero et al
Kolster Oy Ab,
Iso Roobertinkatu 23,
P.O. Box 148
00121 Helsinki (FI)**

(54) **Database techniques for storing biochemical data items**

(57) A database management system for storing a plurality of biochemical data items which originate from one or more external data sources. The database management system comprises a database which stores, for each biochemical data item, an objective identifier (OI) which comprises a set of attributes (143, 144) for characterizing measurable biochemical features of the biochemical data item; and a subjective identifier (SI) which comprises an identity value (112), an identity type value (114, 122) which has an association (118) to the identity value and a reference (128) to a data source, the reference being associated to the identity type value (114, 122).

Fig. 1

Objective identifier:  OI  145  143, 144
= Entity.identified_by + columns specified by it

Subjective identifier:  SI  113  124  135
= Identities.id_string + Identity_types.name + Source.name

EP 1 783 647 A2

**Description**

**BACKGROUND of the invention**

The invention relates to biochemical database, ie, a database for storing biochemical data items which originate from one or more external data sources, such as gene and/or protein sequence data banks. Such external data sources are frequently called databases, but in the context of the present invention, the term 'database' is limited to the inventive database, while the external sources are called data sources or data banks.

Typically, the contents of a biochemical database is not built from scratch. It is more common to import data from external data sources to an internal database within a research facility. This import process is known by an acronym 'ETL', which means Extract data from one or more external data sources, Transform the extracted data and Load it into an integrated database. In this context, an integrated database means one which integrates data from several external sources.

The practice of importing data from several external sources leads to certain problems. For instance, the external data sources have been known to publish information using one set of identifiers and to change the identifiers afterwards. This has severe consequences in the pharmaceutical industry in which data traceability is of paramount importance. In addition, two or more external data sources may use identical identifiers to refer to completely different data items. Or, they may use different identifiers of completely identical biochemical entities.

**Brief description of the invention**

An object of the present invention is to provide a database structure which alleviates the above problems. The object of the invention is achieved by a database which is characterized by what is stated in the independent claims. The preferred embodiments of the invention are disclosed in the dependent claims.

The invention is based on the idea of identifying biochemical data items by a combination of a subjective identifier and an objective identifier. Advantages of the invention include improved data traceability and a reduction or elimination of certain types of errors.

**Brief description of the drawings**

In the following the invention will be described in more detail by means of preferred embodiments with reference to the attached drawings, in which

Figure 1 illustrates an identity structure according to an embodiment of the invention;
Figure 2 shows a concrete example of using subjective identifiers according to an embodiment of the invention;
Figure 3 shows an embodiment in which the entities table includes an additional column for indicating a data source;
Figure 4 is a flow chart illustrating a decision-making routine which is part of an Extract-Transform-Load (ETL) process according to an embodiment of the invention;
Figure 5 shows a truth table which relates to the decision-making routine shown in Figure 4;
Figure 6 shows an alternative, less strict, decision-making routine for an ETL process;
Figure 7 illustrates the operation of the decision-making routine shown in Figures 4 and 5;
Figure 8 illustrates a technique which supports uniqueness constraints in cases wherein two or more external data sources use identical values;
Figure 9 shows a SQL-based implementation for a mapping function;
Figures 10A and 10B illustrate techniques for identifying correspondence between entities originating from different data sources;
Figure 11 illustrates a technique and an example for classifying projects to categories, contextual projects and subprojects;
Figure 12 illustrates a data model used in an embodiment of the invention;
Figure 13 shows a data schema which is based on the data model shown in Figure 12;
Figure 14 illustrates the structure of the various table types shown in Figure 13;
Figure 15 shows a routine for implementing the versioning views block shown in Figure 12;
Figure 16 is a flow chart which illustrates a procedure for tagging fixed tags;
Figure 17 is flow chart illustrating an acceptance procedure based on locking of data; and
Figure 18 is flow chart illustrating an acceptance procedure based on a comparison of message digests.

**Detailed description of specific embodiments**

The following description of specific embodiments of the invention is based on common database terminology, such as tables, columns and rows.

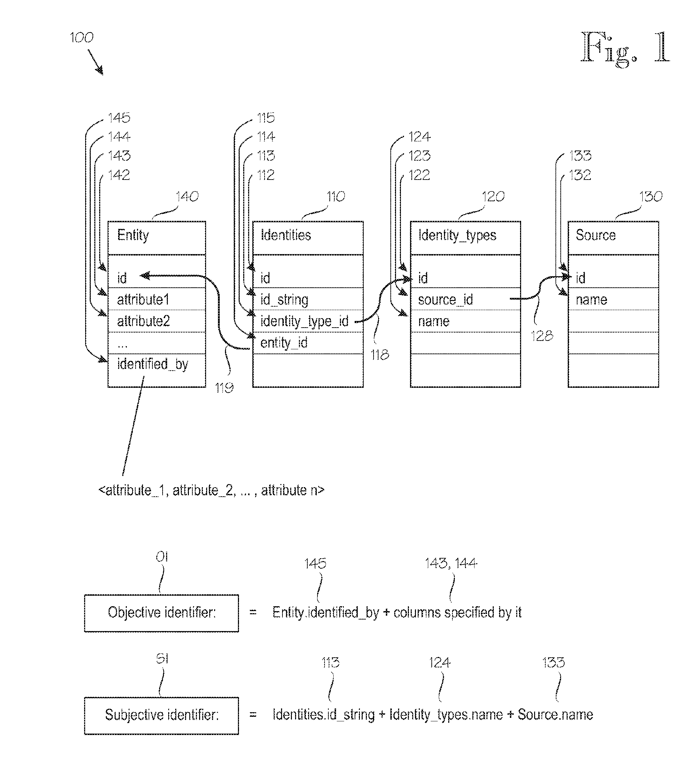

Figure 1 illustrates an identity structure according to an embodiment of the invention. Reference numeral 100 generally denotes a structure of identities. In this embodiment, the structure of identities is built from four tables, namely an *Identities* table 110, an *Identity_types* table 120, a *Source* table 130 and an *Entity* table 140.

In this implementation, the columns of the *Identities* table 110 include id 112, *id_string* 113, *identity_type_id* 114 and *entity_id* 115. The columns of the *Identity_types* table 120 include *id* 122, *source_id* 123 and name 124. The columns of the *Source* table 130 include id 132 and name 123. The columns of the *Entity* table 140 include id 142, a number of attribute columns of which two are shown *(attribute1* 143 and *attribute2* 144), and an *identified_by* column 145.

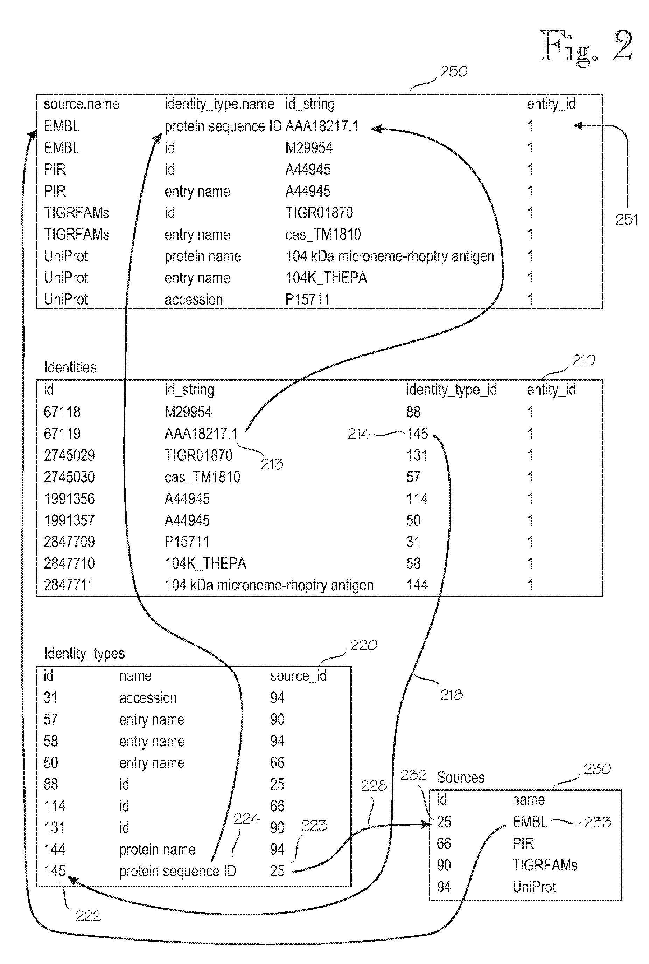

Column *identity_type id* 114 in the *Identities* table 110 is a foreign key 118 to column id 122 in the *Identity_types* table 120 and a relation 119 to column id 142 in the *Entity* table 140. Column id 122 in the *Identity_types* table 120, in turn, is a foreign key 128 to column id 132 in the *Source* table 130. The concrete example shown in Figure 2 will clarify the meaning of these fields and relations.

In this implementation, the objective identifier OI is formed by the *identified_by* column 145, and those attribute columns which are specified by the *identified_by* column 145 (the *Entity* table 140 may comprise further attribute columns which are not specified by the *identified_by* column 145). In this example, let us assume that the *identified_by* column 145 specifies columns *attribute1* 143 and *attribute2* 144.

The subjective identifier SI comprises, at a minimum, the columns joined by the two database relations 118 and 128, ie, the columns id 112 in the *Identities* table, id 122 in the *Identity_types* table 120 and id 132 in the *Source* table 130. For the benefit of human users, the subjective identifier SI should preferable comprise also the *id_string* 113. Figure 1 shows a preferred implementation in which the subjective identifier SI comprises items *'Identities.id_string'* 113, *'Identity_ types.name'* 124 and *'Source.name'* 133.

Figure 2 shows a concrete example of using the subjective identifiers according to an embodiment of the invention. In Figure 2, most reference numerals smaller than 250 are instances (practical examples) of their corresponding elements in Figure 1, such that item 2xx in Figure 2 is an instance of the corresponding item 1xx in Figure 1. For instance, item 210 is an instance of the *Identities* table 110 shown in Figure 1, an so on.

Reference numeral 250 denotes a data view which is generated from the *Identities* table 210, *Identity_types* table 220 and *Sources* table 230. Line 251 of the data view 250 indicates the following information: "data source 'EMBL' contains a column 'protein sequence ID' which has a value of 'AAA18217.1' for entity #1". This information is derived from the tables 210, 220 and 230 as follows. Item 213 in the *Identifies* table 210 contains one row of the column *id_string* 113 shown in Figure 1. Its value is the protein sequence ID , namely 'AAA18217.1'. Item 214 in the *Identities* table 210 contains one row of the column *identity_type_id* 114. Its value is '145'. Reference numeral 218 denotes an example of the relation 118 to column id 122 in the *Identity_types* table 120. The relation 218 references item 222 in the *Idenlify_ types* table 220. The same row in the *Identity_types* table 220 contains item 234, which indicates 'protein sequence ID' as the *idenfity_type.name* in the view 250. Finally, the row of the referenced item 222 indicates '25' as the value of *source_id* 225. From the *source_id* 225 there is a relation 228 to item 232 in the *Source* table 230. The row of item 232 in the *Source* table 230 indicates 'EMBL' as the value of *name* 233. The *name* item 233 is used as *source.name* in the data view 250.

It is apparent from Figure 2 that the data view 250 is very flexible. Not only does the data view 250 indicate a value ('AAA18217.1') for the protein sequence identifier but also the name used for this value in the external data source, namely 'protein sequence ID'. This name is not hard-coded in the *Identities* table 210 but derived via relation 218 from the *Identity_types* table 220. Likewise, the name of the data source is derived via relation 228 from the *Sources* table 230, whereby the data structure shown in Figure 2 is not only flexible but provides improvements to data traceability as well.

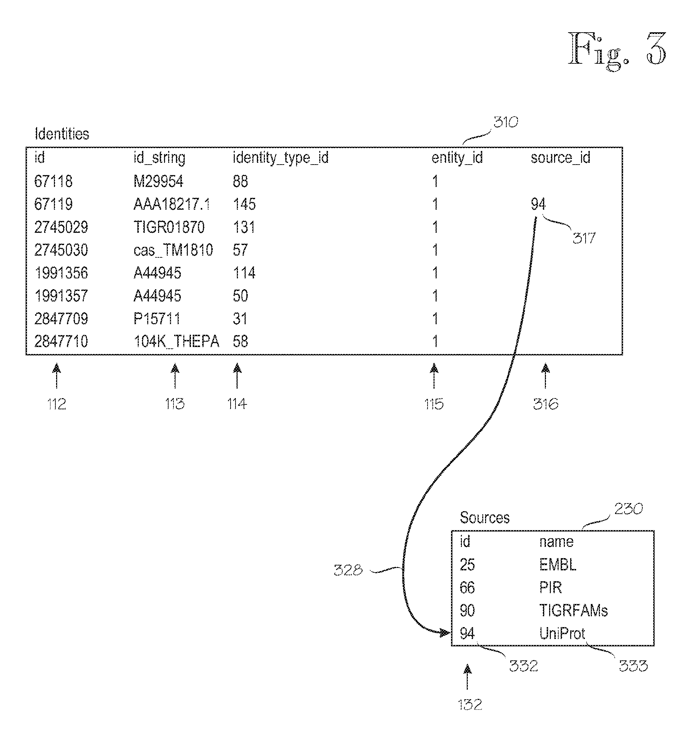

Figure 3 shows an embodiment in which the *entities* table, denoted here by reference numeral 310, includes an additional *source_id* column 316 for indicating a data source. Item 317 denotes one row of column 316 for the row which was described in connection with Figure 2. In this embodiment, there is a relation 328 from column 316 to the id column in the *Source* table 230 (item 132 in Figure 1). The relation 328 is a direct relation from the *Identities* table 310 to the *Sources* table 230, unlike the indirect relations 218, 228 via the *Identity_types* table 220. In the example shown in Figure 3, item 317 has a value of '94'. This value links to item 332 in the *Sources* table 230. The corresponding *name* column contains item 333, whose value is 'UniProt'. The information provided by the relation 328 and items 317 and 333 means that row #2 *Identities* table 310, which contains item 317, has been obtained from data source 'UniProt'. This embodiment further improves data traceability and quality. Quality of data is improved because first-hand information can be separated from second -hand information. Suppose that data source X reports that data source Y has an identity Z for entity W. This is an example of second -hand information, which tends to be less reliable than first-hand information in which the data source Y itself reports having an identity Z for entity W. When data sources report their own identities, such information is usually verified by foreign keys. On the other hand, when data sources report second-hand information, any error correction is usually limited to correcting errors spotted by human users. The first-hand information also implicitly indicates the data source version in which the first-hand information exists, ie, the data source version currently being integrated into the database. Second-hand information lacks such an indication. As a result, the *'source.id'* item 123 (see Figure 1) must point to some generic version of the external data source.

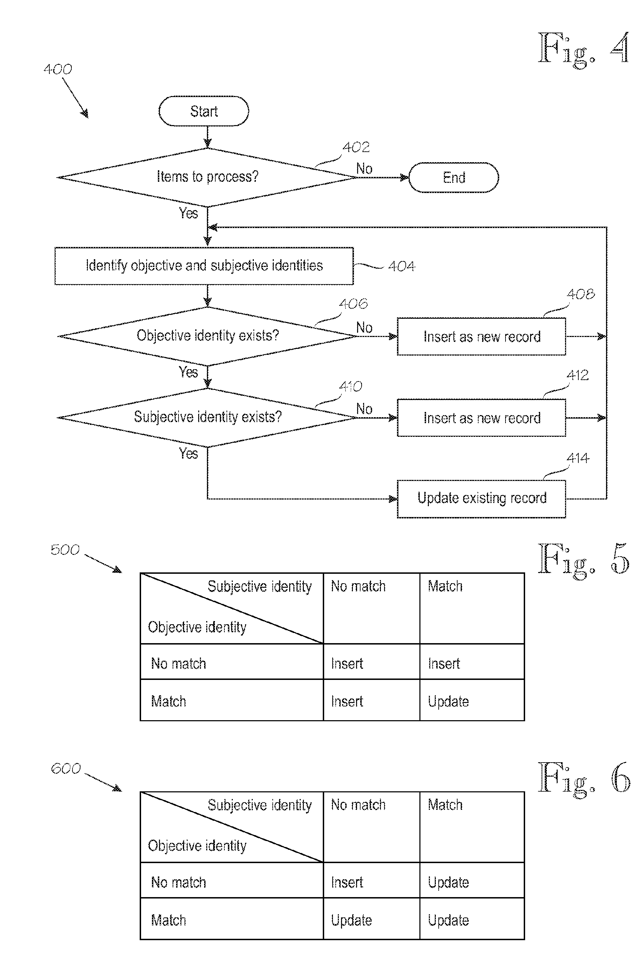

Figure 4 is a flow chart illustrating a decision-making routine 400 which is part of an Extract-Transform-Load (ETL) process according to an embodiment of the invention. In step 402 the ETL process checks if all items have been processed or if there are more items to process. If all items have been processed, the process ends. Otherwise the ETL process identifies in step 404 the objective identifier OI and subjective identifier Si of a biochemical data item which is being obtained from an external data source. In step 406 the ETL process checks whether the objective identifier OI already exists in the database. If not, the data item is inserted as a new record in step 408. If the objective identifier OI already exists in the database, the ETL process checks in step 410 whether the subjective identifier SI already exists in the database. If not, the data item is inserted as a new record in step 412. Only if the objective identifier OI and the subjective identifier SI both exist in the database, the ETL process executes step 414 in which the data item being processed is used to update an existing record in the database. In other words, if the database contains no record whose objective identifier OI and subjective identifier SI match those of the data item being processed, the data item is inserted as a new record.

Figure 5 shows a truth table which is equivalent to the loop 404 - 414 in the decision-making routine 400 shown in Figure 4. In the truth table 500, 'No match' means that the subjective or objective identifier of a data item being processed matches the respective identifier in none of the existing records in the database. 'Match' means that the subjective or objective identifier of a data item being processed matches the respective identifier in at least one existing record in the database. The truth table 500 indicates that only if both identifiers match, is the data item used for updating an existing record, and otherwise it is inserted as a new record.

Figures 4 and 5 show a very strict decision-making routine which inserts any variations of data as new records. This embodiment is particularly applicable in pharmaceutical research wherein very small variations can be critical.

Figure 6 shows an alternative embodiment in which, if either of the subjective or objective identifiers matches at least one existing record, the data item is used for updating the existing record. The data item is inserted as a new record only if there is no match between either identifier and any of the existing records. This embodiment results in more integrated data but suppresses variations of data.

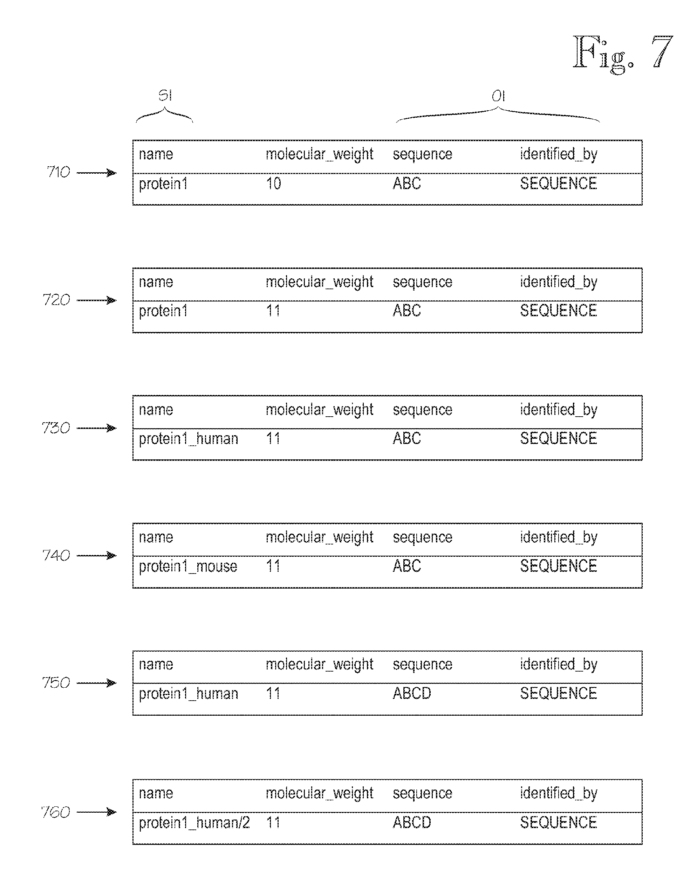

Figure 7 illustrates the operation of the decision-making routine 400 or 500 shown in Figures 4 and 5. reference numeral 710 denotes an entity in a data source. The parameters of the entity 710 include *name, molecular_weight, sequence* and *identified_by.* The *name* element is part of the subjective identifier SI. The *identified_by* column was described as item 145 in connection with Figure 1. The contents of this item is *'sequence',* which means that the *sequence* attribute is used as the objective identifier OI. The *molecular_weight* is an example of an attribute which is not indicated by the *identified_by* column and, consequently, is not part of the objective identifier OI.

The first time the decision-making routine 400 or 500 encounters the entity 710, it naturally inserts the entity 710 as a new record. Next, let us assume that the external data source provides an updated version 720 of the entity 710. In the updated version 720, the *molecular_weight* has been corrected to 11. During the next execution of the decision-making routine 400, the objective identifier OI, ie, the *sequence* item 'ABC', in record 720 matches the corresponding item in 710 which has been stored in the database. The question in step 406 is answered in the affirmative, and the logic proceeds to test 410. The question in step 410 is also answered in the affirmative because the subjective identifiers SI, ie, the contents of the *name* column in the records 710 and 720, are identical. Accordingly, the logic proceeds to step 414 in which the existing record 710 is updated by the contents of the record 720. Specifically, the value of the *molecular_weight* column is updated from '10' to '11'.

Assume now that the external data source is updated such that the record 720 is split into two records 730 and 740, which relate to humans and mice, respectively. The record 730 has 'protein1_human' in its *name* column, while record 740 has 'protein1_mouse'.

Next time the decision-making routine 400 is executed, the currently existing record in the database is record 720 and the record being processed is record 730. The objective identifiers OI match *(sequence = 'ABC'),* but the subjective identifiers SI don't ('protein1_human' ≠'protein1'). Thus the question in step 410 is answered in the negative and record 730 is inserted as a new record in step 412. This also happens when record 740 is processed, because there is no existing record which matches the subjective identifier value of 'protein1_murine'. Record 740 is thus inserted as a new record. The database now contains records 720, 730 and 740.

In the last phase shown in Figure 7, let us assume that the external database updates record 730 to record 750. Specifically, the contents of the *sequence* column has been updated to 'ABCD'. Next time the decision-making routine 400 is executed, it checks in step 406 whether the database has a record which matches the objective identifier OI of record 750, which contains the *sequence* column with a value of 'ABCD'. There is no match, and the record 750 must be inserted as a new record in step 408. However, inserting the record 750 as a new record creates a problem because the *name* column has a uniqueness constraint applied to it. In other words, the *name* column should not contain duplicate values. Accordingly, the insertion routine 408 must ensure that the *name* column in the record being inserted has a unique value. In this example, the insertion routine 408 applies a slash and a running number '2' to the value of the *name* column. The inserted record is shown as item 760. At this point the database contains records 720, 730, 740 and 760.

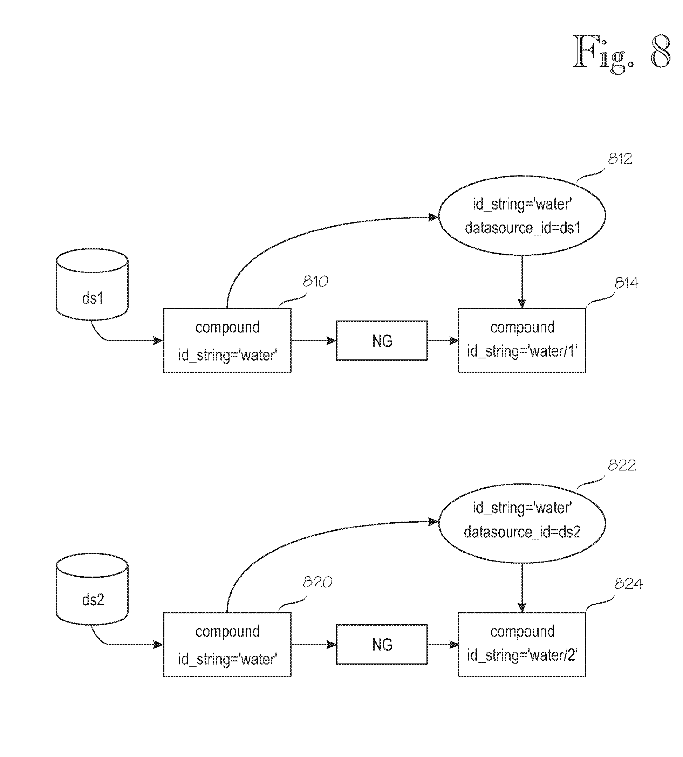

Figure 8 illustrates a technique which supports unique constraints in cases wherein two or more external data sources use identical values. In the example shown in Figure 8, the ETL process integrates compounds from two data sources ds1 and ds2. Both data sources contain references to 'water' but they assign different meanings to it. In one of the data source, water means any form of water as it exists in nature, such as in oceans. In the other data source, the term "water" is restricted to chemically pure water $H_2O$. It is clear that the database needs two different records to represent the two meanings of water. But changing the name of "water" to something else, at least in one of the records, for allowing uniqueness constraints, is confusing to human users who are used to referring to all forms of water by the name "water".

These problems are solved by the embodiment shown in Figure 8, in which a name generator routine NG generates the compound names (or any other names with uniqueness constraints) either automatically or on demand. The name generator routine NG adds an appendage, such as a prefix or postfix, to mapped names. The prefix or postfix can be an identifier of the data source, a running number or the like. In the case of entities 810 and 820 extracted from respective data sources ds1 and ds2, the name generator routine NG applies respective postfix definitions 812 and 822 for compound "water". The mapped compound names are denoted by reference numerals 814 and 824, respectively. The postfix definitions 812 and 822 relate to stored identities in the database. In this example, the name generator routine NG ignores the *'identity_type'* items in the postfix definitions 812 and 822 and only appends a running number. It is not necessary to incorporate the name of the original data source to the compound names 814 and 824, as long as the compound names 814 and 824 fulfill the unique constraints.

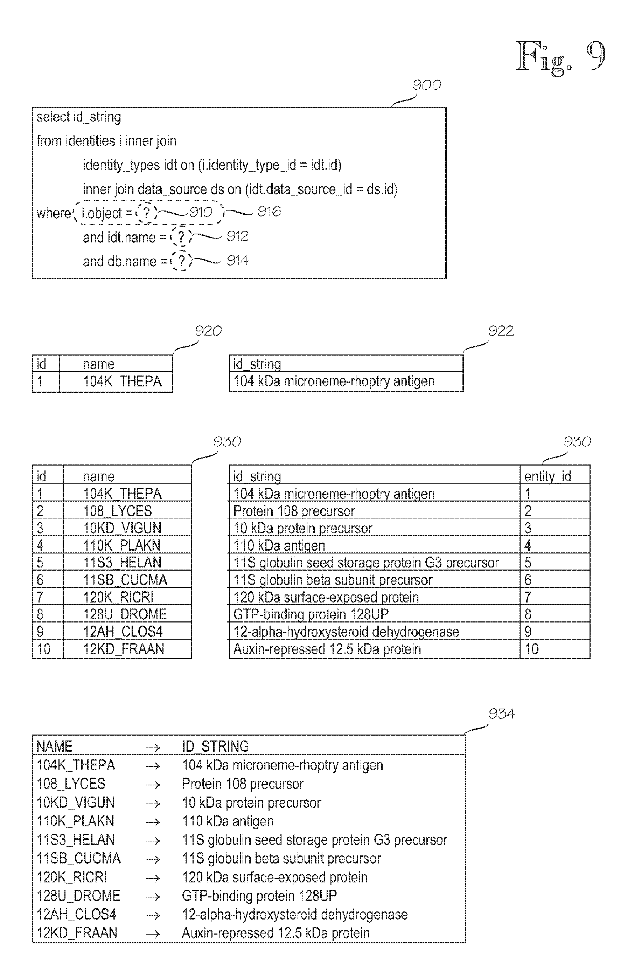

Figure 9 shows a SQL-based implementation for a mapping function MF. Mapping of namespaces allows users and applications to operate using names which differ from the names stored internally or used in the original data sources. Reference numeral 900 denotes a routine in SQL code which performs mapping from one namespace to another. The question marks 910, 912 and 914 act as placeholders and will be replaced by concrete character strings, depending on the names to be mapped. Reference numeral 916 denotes a search criterion which restricts the mapping to objects which meet the condition that i.object equals the string which replaces the first placeholder 910.

Reference numeral 920 denotes an entity which is to be mapped to 'protein name' of data source UniProt by using the routine 900 and the data shown in Figure 2. The first placeholder 910 will be replaced by '1', which is the id field of the entity 920. The second placeholder 912 will be replaced by 'protein name', and the third placeholder 914 will be replaced by 'UniProt'. Reference numeral 922 denotes the result of the mapping, ie, the mapped identity.

The routine 900 can be used to map all entities by removing the search criteria 916. Reference numerals 930 and 932 denote, respectively, a first and a second name space. Reference numeral 934 denotes a table which shows the result of the mapping.

The mapping function shown in Figure 9 can be used to map names from one name space to another. For instance, the name spaces could be different human languages or naming conventions of equipment manufacturers. Or, biochemical entity names could be mapped to trade names of pharmaceutical products.

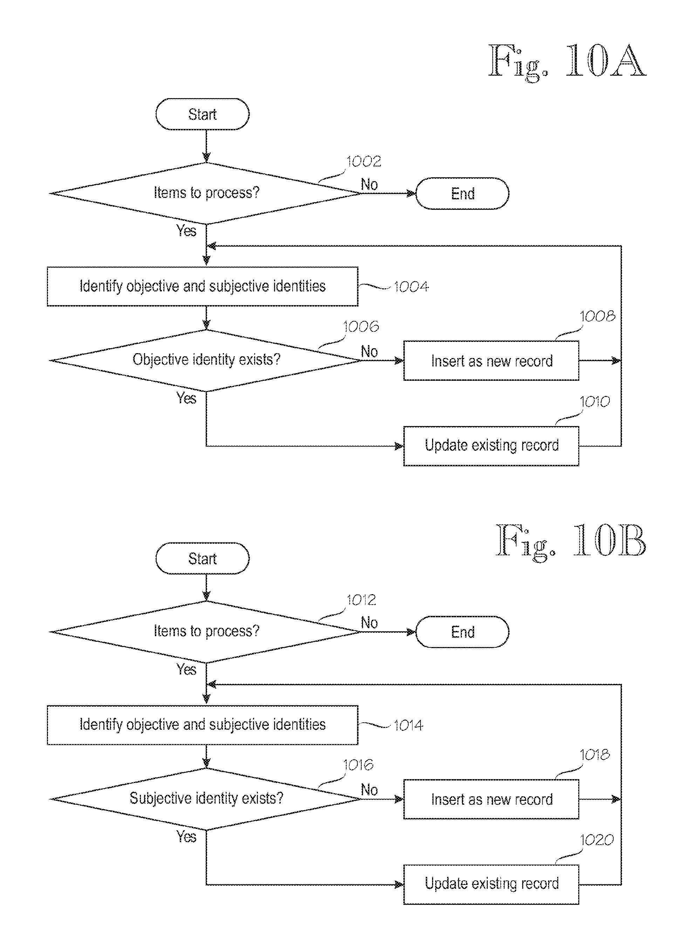

Figure 10A and 10B illustrate techniques for identifying correspondence between entities originating from different data sources. When extracting data from two or more external data sources to an integrated database, the external data sources can be overlapping, complementary or a combination of both. In case of overlapping data sources, the objective identifier OI can be used for finding correspondence between entities originating from different data sources. For example, two data sources containing data about proteins may use different names for the same human protein. If the data sources contain proteins identified on the same level of generality, the routine shown in Figure 10A can be used. An illustrative but non-restricting example of proteins identified on the same level of generality is two data sources in which proteins are identified by sequence. The routine shown in Figure 10A is a simplified version of the routine shown in Figure 4, and a detailed description is omitted. If the external data sources are complementary, the routine shown in Figure 10B can be used. This routine is also a simplified version of the routine shown in Figure 4, and a detailed description is omitted.

**Data version management**

Figure 7 and its associated description disclosed a need for systematic version management for data. In the hypothetical, but not too far-fetched, scenario shown in Figure 7, externally-originated protein *'protein1'* had its name, sequence and molecular weight changed. In situations like this, it is important to have up-to-date information available, but biochemical research institutions cannot use simple update processes which merely replace old data with new. Responsibility considerations require that all research data and results must be traceable to their origins. This is particularly important in the development of medicaments. Old information is normally locked from new projects but kept available to existing projects and for record-keeping. For example, an experiment which begins by using one set of data should normally end by using the same set of data. Thus there is a need for systematic version management which is able to present stable versions of data to users and applications.

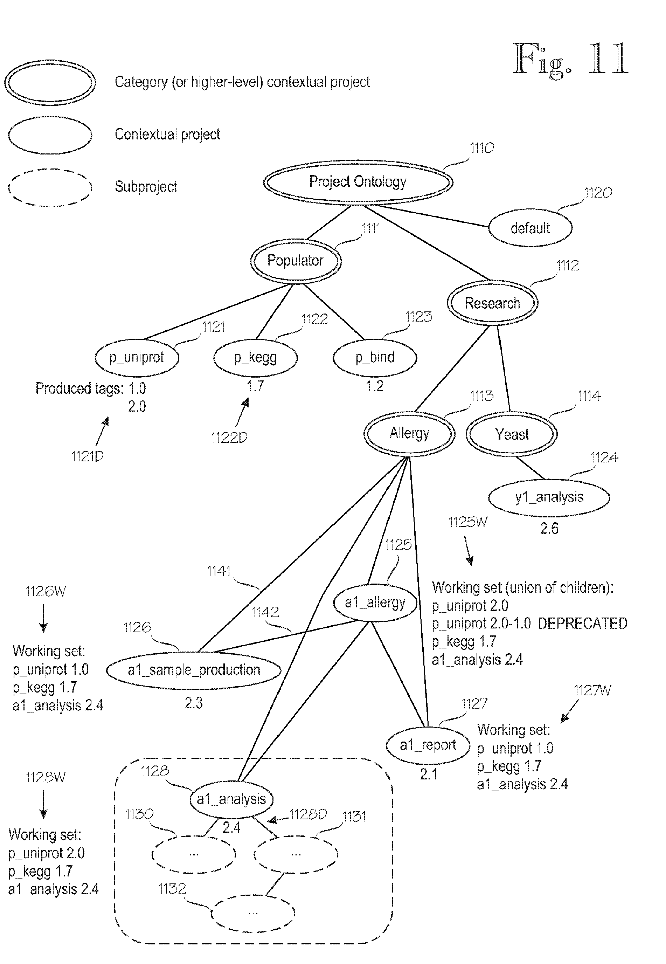

Figures 11-16 relate to embodiments which implement systematic version management. Specifically, Figure 11 illustrates a technique and an example for classifying projects to categories, contextual projects and subprojects. In this embodiment, the categories, contextual projects and subprojects are organized in a structure which is not strictly a tree structure although it resembles one. Figure 11 shows an embodiment in which the topmost hierarchical level is category, but the invention can be implemented without the concept of category, in which case any categories are merely replaced by higher-level contextual projects, ie, contextual projects which are parents to other contextual projects. In this example, the topmost category is *'Project ontology'* 1110. Figure 11 shows two of its child categories, namely *'Populator'* 1111 and *'Research'* 1112. The *'Research'* category 1112, in turn, has two child categories, namely *'Allergy'* 1113 and *'Yeast'* 1114.

Categories may also be parents to contextual projects. A contextual project is a project which has a context. The context comprises a working set of data. The concept of a working set constitutes an aspect of version management for data, as will be described later in more detail. Contextual projects are actual research projects into which users can log in. They produce data for other projects. Naturally, it is the fruitful cooperation between a human research team and an information management system which 'produces' data, but in terms of version management, 'production' of data refers to tagging a certain contextual project as the origin of any piece of information, whereby the origin of that information is traceable.

A contextual project may be a parent to another contextual project and/or to a subproject. In this example, contextual project *'a1_allergy'* 1125, which is a child of category *'Allergy'* 1113, has three contextual projects as its children, namely *'a1_sample_production'* 112fi, *'a1_report* 1128 and *'a1_analysis'1128,* all of which are also children of category *'Allergy'* 1113.

Figure 11 shows an embodiment in which the organization of contextual projects is not strictly tree-like in structure. This means that parent-child relations, ie, the associations between contextual projects, cannot be implemented by merely having a "parent" identifier in each contextual project. Instead, the associations between contextual projects can be implemented by means of dedicated data records, each of which forms an association between one child project and one parent project. For instance, records 1141 and 1142 both have *'a1_sampleyroduction'* 1126 as the child project, but the former has category (or higher-level project) Allergy' 1113 as the parent, while the latter has *'a1_allergy'* 1125 as the parent.

The contextual project *'a1_analysis'* 1128 is parent to two subprojects 1130 and 1131, of which the latter has a subproject 1132 of its own. The names of the subprojects are hidden to reduce clutter in Figure 11. Subprojects can be used to divide big projects into smaller parts without the need to set up a new contextual project for each part. Each subproject always has a well-defined parent project. Users cannot log in to subprojects. All data produced in a subproject belongs to its parent contextual project, which is why the subprojects of each contextual project should form a strict tree-like structure, with the contextual project being the root node of the tree.

Each contextual project has a context which comprises a working set of data. Figure 11 shows four contextual projects, namely 1125, 1126, 1127 and 1128, for which a working set of data is shown. The working set of a contextual project is denoted with a reference sign which is the reference numeral of the contextual project with a 'W' (for working set) appended to it. For instance, reference sign 1125W denotes the working (data) set of contextual project 1125.

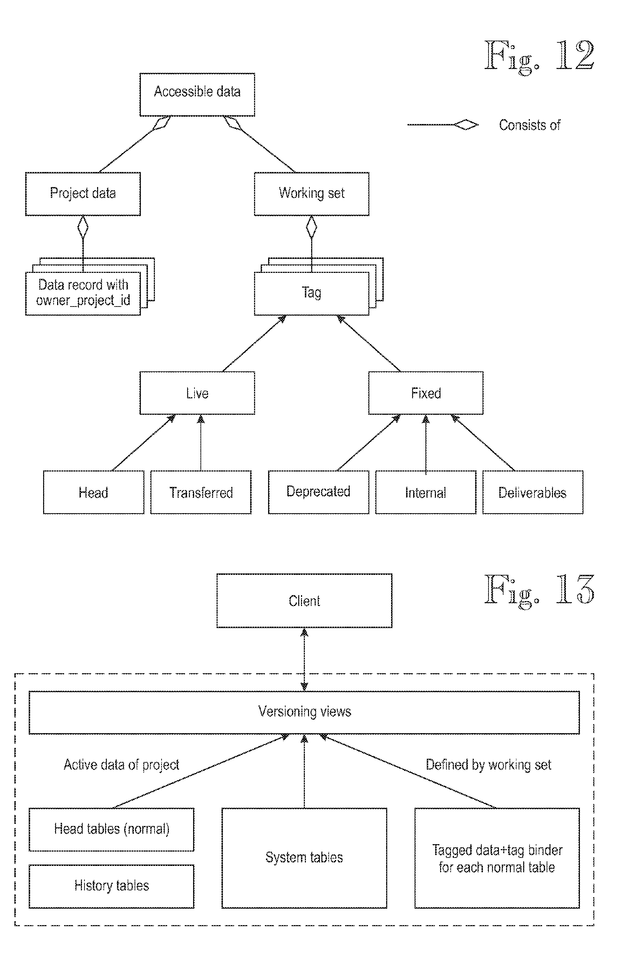

Figure 12 illustrates a data model used in an embodiment of the invention. The following description relates to both Figures 11 and 12 such that Figure 12 shows a generic data model, while Figure 11 shows a concrete example.

Thus the data accessible to a contextual project may contain two types of data, namely data actually "owned" by the project and data "borrowed" from other contextual projects. A contextual project "owns" (or more technically speaking: is marked as the owner of) the data produced in the contextual project and all of its subprojects. All data belongs to some specific contextual project. In Figure 11, data owned by contextual project is denoted by the reference numeral of that contextual project and a 'D' (for data) appended to it. For example, reference sign 1121D denotes data owned by contextual project 1121. In this example, such data comprises two tagged versions (1.0 and 2.0) of *p_unipmt* data.

For example, the data records may be created with a data entry application, workflow editor, pathway editor or project editor. In one implementation, each data record has a column such as *'owner_project_id'* which specifies the owner of the data record. As a result of a user logging in to a specific contextual project, all data records produced in that session will have their *'owner_project_id'* fields pointing to the owner project. In an alternative implementation, there may be separate owner records which associate data records to contextual projects. This feature may be implemented in each specific application or data editor. Or, the feature may be implemented as a process in a database server, in which case the applications/data editors do not have to implement the owner-marking process which is transparent to them. A contextual project may only modify its own project data, in contrast to project data from other contextual projects which is only accessible in read-only mode via a working set.

Thus all data accessible to a contextual project is not necessarily owned by that contextual project as project data. Instead a contextual project may use data owned by other contextual projects. A contextual project's working set of data is a list of tags which define the data that is accessible (in read-only mode) to the project. Typically these tags contain deliverables of projects such as population and derivation projects.

A tag is a snapshot of the data of a contextual project at a specific instance, such as at a specific moment of time. Tags are information structures by means of which a contextual project can publish its data such that other contextual projects can include the data in their working set. As used herein, "publish" does not necessarily mean making the data available to the general public. Instead it means a technique for making data available to other contextual projects and their users, provided that the users have the proper privileges to log in to such projects. A typical tag contains data from a single contextual project but it can also collect data from multiple projects. For example, the ability to tag data belonging to a plurality of categories is beneficial in building complex data sets, such as evolution sets of the populated data, data imported by the Extract-Transform-Load (ETL) process.

There are two types of tags, namely live and fixed. A live tag is one whose contents may change. In contrast, the contents of fixed tags remain unchanged and cannot be edited anymore. The live tags are further classified into two types, namely HEAD and TRANSFERRED. The fixed tags, in turn, are further classified into three types, namely DEPRECATED, INTERNAL and DELIVERABLES. These types will be further described in connection with Figure 13.

In Figure 11, data version management can be seen in the fact that projects 1126 and 1127 use version 1.0 of *p_uniprot* data, project 1128 uses version 2.0, while project 1125 uses version 2.0 and version 1.0, of which version 1.0 is tagged as DEPRECATED (=marked obsolete).

Figure 13 shows a data schema which is based on the data model shown in Figure 12. A client application does not access data directly but via a versioning layer which consists of versioning views to the data. The views provide a consistent view to the database, wherein all foreign keys and unique constraints are followed as specified in the applicable information model.

Head tables contain the data of head tags. They contain the most recent version of data records. Head tables are normal database tables, as if no version management was implemented. History tables resemble head tables but they have two extra columns. History tables not visible to applications, as are masked by an abstraction layer added on top of the raw data, ie, data consisting of actual database records. The abstraction layer can be implemented by using read-write and read-only views.

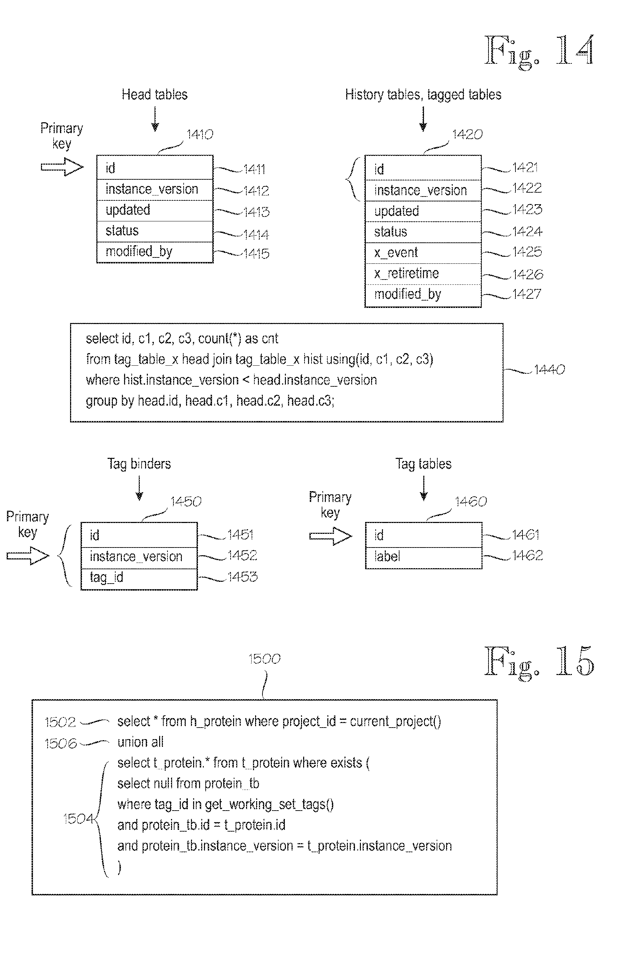

Figure 14 illustrates the structure of the various table types shown in Figure 13. Reference numeral 1410 denotes the structure of head tables. The primary key of head tables is the identifier field *'id'* 1411. *Instance_version* 1412 is a field which indicates the version of the record in question. It begins at a predetermined number, such as 1 (or 0) and is incremented each time the record is updated. *'Updated'* field 1413 contains a time stamp which indicates when the record was updated (or created if it has not been updated). *'Status'* field 1414 may contain any of the application-specific status values plus DELETED, which indicates that the record is no longer active. Unique constraints of head tables include the primary key *'id'* 1411 and any unique columns which are defined in the information model. Unique columns cannot be modified; instead a new record must be created and the old record is copied to a history table. The final field (column) 1415 indicates the user who performed the last modification.

Reference numeral 1420 denotes the structure of history tables. The four first columns 1421 - 1424 correspond to columns 1411 - 1414 of head tables. However, none of the columns of history tables can be used alone as a primary key because they contain every version of every id. Instead the primary key of history tables is the combination of *id* 1421 and *instance_version* 1422.

When a record is inserted into a head table, it is copied by database triggers to the history table and the value of the *x_event* field 1425 in the history table is set to 'INSERT'. The *x_retiretime* field 1426 remains at 'NULL' because the current version is the latest version of that record. When a record is updated in the head table, the new version is copied to the history table such that the *x_event* item 1425 is set to 'UPDATE'. Again, the *x_retiretime* 1426 remains at 'NULL' because that version is the latest version of that record. But the previous version of that record in the history table is updated so that its *x_retiretime* 1426 is set to the current time. When a record is deleted, the old version (before the actual deletion) is copied to the history table and its *instance_version* field 1422 version is incremented by one. The *x_retiretime* 1426 field remains at 'NULL' because that version is the latest version of that record. The previous version of that record in the history table is updated so that its *x_retiretime* field 1426 is set to the current time. Field 1427 indicates the user who performed the last modification (insert, update or delete operation).

Thus the history tables store a full audit trail in them. The audit trail can be retrieved by querying the history tables directly. There is no need to implement any views to the history tables. Instead the history tables can be accessed directly. In addition, history tables can be used in "point in time" -type of queries. The updated column contains the time stamp when the record has become active and *x_retiretime* when it became inactive. The "point in time" queries can be implemented just by adding the following line to the query for each joined table:

$$:time > updated \ AND \ :time <= COALESCE(x\_retiretime, :time)$$

In the above query specifier, the term *:time* is the time of interest and "coalesce" is a function which selects *:time* if *x_retiretime* is null.

The structure of tagged tables can be identical to the structure of history tables, which is denoted by reference numeral 1420. Tagged tables differ from history tables in respect of their unique constraints. As regards unique constraints, tagged tables are problematic because they can contain different versions of the same records of head tables. Thus the unique constraints applied to head tables are not applicable to tagged tables. According to an embodiment of the invention, uniqueness throughout history is guaranteed by means of an extra table or materialized view. Reference numeral 1440 denotes an SQL query fragment which can be used to guarantee uniqueness. *'c1'*, 'c2' and *'c3'* are unique columns in a head table. The result of the view is then materialized by using the functionality of the relational database engine and a unique constraint is imposed on the materialized result columns *'c1'*, *'c2'*, *'c3'* and *'cnt'*. If the unique constraint does not have to be applied across the entire history, then the unique index in the head table can simply be added as a non-unique index to the tag table.

Indexing in tagged tables can be optimized for large/complex queries and rare batch inserts. For example, bitmap indexes, if provided by the database engine, are advantageous because only batch inserts are performed when a project is tagged, and no single-row updates, inserts or deletions are needed.

Reference numeral 1450 denotes the structure of a tag binder (table). Tag binders connect a record in a tag table to a specific tag. Tag binder tables have three columns: *'id'* 1451, *'instance_version'* 1452 and *'tag_id'* 1453. *'id'* and *'instance_version'* columns 1451, 1452 form a foreign key to the corresponding tagged table. Column *'tag_id'* 1453 is a foreign key to the corresponding tag. The primary key of the tag binder is compound of all columns 1451 - 1453.

Reference numeral 1460 denotes the structure of tag (tag tables). A tag table contains at least two columns: 'id'l 1461 and *'label'* 1462, of which the *'id'* column 1461 is the primary key.

Figure 15 shows a routine for implementing the versioning views block shown in Figure 12. Versioning views emulate the tables prior to implementing or enabling versioning. In the following, versioning will be described with reference to a hypothetical table named *'protein'*. In one embodiment, the versioning functionality involves renaming the original table named *'protein'* to *'h_protein'* and adding three more tables, namely a corresponding history table *'x_protein'*, tagged table *'t_protein'* and tag binder *'protein_tb'*. A *'protein'* view is also added. In case of data manipulation, the tasks of versioning views include:

- updates and inserts to versioning views are forwarded to the head table;
- converting deletions of head table records to setting the corresponding status field to 'DELETED';
- converting inserts, updates and deletions to inserts into history table and setting the *'x_event'* column according to the relevant data manipulation event.

The versioning views assume that when a user has an open session, he or she has been logged in to some project. The versioning view selects head records from the current project and all records from tagged tables which are part of the working set of the current project.

Reference numeral 1500 denotes a routine (in pseudocode) which implements a view *protein'*. Line 1502 retrieves from the history table *'h_protein'* all records which belong to the project whose id is 'current project'. In other words, this line retrieves the project's own data. The bulk of the routine 1500, denoted by reference numeral 1504, retrieves the project's working set from the tagged table *'t_protein'*. Line 1506 forms a union of the project's own data and working set (other projects' data). The union operation is very simple; duplicate rows do not need to be filtered out because there aren't any.

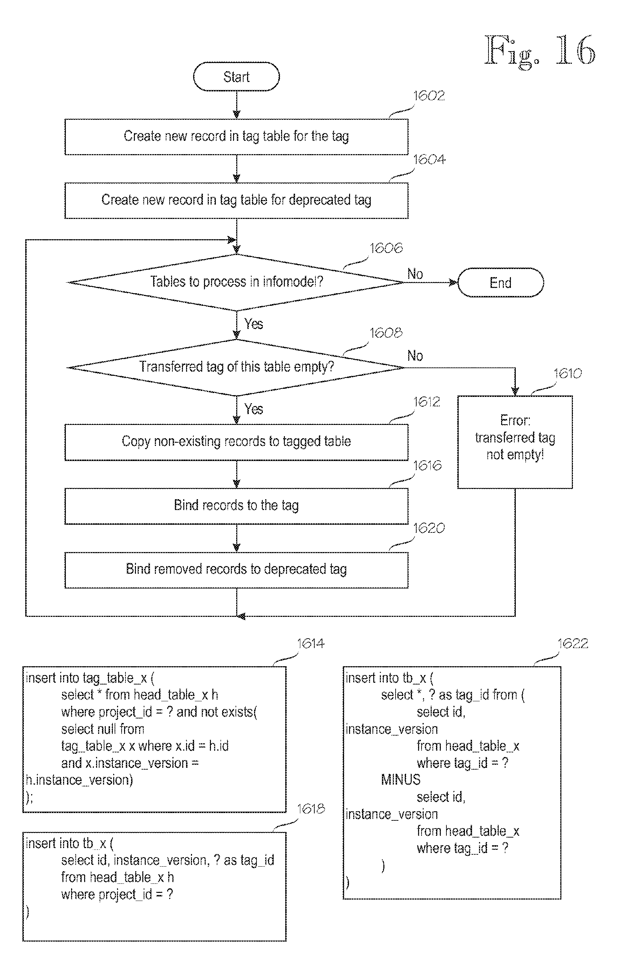

Figure 16 is a flow chart which illustrates a procedure for tagging fixed tags. Tagging involves adding head records in a project to a label The records to be tagged can be selected on the basis of a rule. If no rule is applied, all records in the head table will be processed. The tagging process locks the data from further alterations.

In step 1602 a new record is created in the tag table for the tag. In step 1604 a new record is created in the tag table for a deprecated tag. The purpose of this step is to achieve backwards compatibility of data. For instance, a project P may have used a protein X which is deleted in a new tag. If the project P refers directly to the new tag, the data for protein X becomes corrupted, because the working set of project P does not contain protein X. In order to avoid such a corruption of data, a deprecated tag containing any deleted objects is generated automatically. By means of the deprecated tag, the project P can use the new tag and the corresponding deprecated tag and sort out what to do with entities which refer to the protein X. When all references to the protein X have been eliminated, the deprecated tag can be deleted from the working set.

Step 1606 initializes a loop for processing each table in the information model. The process ends when all tables have been processed. Step 1608 contains a test concerning whether the transferred tag of this table is empty. If the transferred tag is not empty, an alarm is given in step 1610. Each contextual project has a transferred tag into which will be copied any objects whose ownerships are transferred. For instance, a researcher may create an annotation beforehand, and if the same annotation is detected in an ETL process, it must be transferred from the project of the researcher to the ETL project, because a single piece of data cannot coexist in two projects (the *'owner_id'* can only have a single value). When the ETL process becomes the owner of the researcher's object, it must be inserted into the researcher's project as a transferred tag lest the researcher's project becomes corrupted. When the researcher tags his/her own project, he/she must first resolve any conflicts caused by the transfer by using the tag of the ETL process and delete the annotation from the transferred tag. This chain of events ensures fulfilment of the unique criteria.

In step 1612, non-existing records are copied to the tagged table. As used herein, a non-existing record means a record which has existed in a previous version (tag) but not in the current one. For instance, the non-existing records can be selected on the basis of the *'id'* and *'instance_version'* columns 1451, 1452. Reference numeral 1614 near the bottom of Figure 16 denotes an SQL routine for implementing step 1612. The question mark is a placeholder for the *'id'* item of the project to be tagged.

In step 1616, records are bound to the tag. Reference numeral 1618 denotes an SQL routine for implementing step 1616. The first question mark is a placeholder for the *'id'* item of the tag created in step 1602, while the second question mark is a placeholder for the 'id' item of the project to be tagged.

In step 1620, removed records are bound to deprecated tags. Reference numeral 1622 denotes an SQL routine for implementing step 1620. This routine queries those records which existed in the previous tag but do not exist in the current one. The first question mark is a placeholder for the *'id'* item of the deprecated tag, the second one is a placeholder for the previous tag, while the last question mark is a placeholder for the current tag created in step 1602.

As stated above, the procedure in Figure 16 relates to tagging fixed tags. Live tags are simpler in the sense that they are the records which exist in the head tables and do not require additional tagging or similar maintenance.

Transferred tags are slightly more complex. Transferred tags are used to resolve conflicts in unique constraints between projects. Such conflicts may arise when populating data from an external data source and another project has already populated some data from the same data source. In this case the populating project can transfer the data from the other project to itself because only one project should be marked as the owner of the data.

**Acceptance/signature procedure**

The data version management described above, in connection with Figures 11 - 16, is preferably enhanced by an acceptance procedure which supports signing tagged data sets with digital signatures. Digital signatures are frequently used to verify the authenticity of computer-related files, such as digital documents or software executables. But it is not immediately obvious how digital signatures can be applied to published (tagged) sets of database records. One way to apply digital signatures to published data sets is to extract the records to be published into digital files, which are then signed, but this technique has the drawback that such signed files do not appear as database records to other projects which may need them. In other words, the digitally signed files are not processable by conventional database operations. Therefore a need exists for digitally signing a tagged set of database records.

The inventive technique of digitally signing a tagged set of database records is based on the idea that that data is exported from the database and streamed but not stored into a file, at least not permanently. The streamed data is then signed, whereby it is possible to verify afterwards that the data has not been altered after the signing and that a certain person or organization has really consented to accept the contents of the data stream. This technique involves the problem that the database must be able to reproduce the data stream in identical versions, bit for bit, for the signing and verification processes. This is a clear departure from conventional techniques in which digital signatures are applied to and verified against concrete documents. In one illustrative embodiment, the inventive signature technique involves the following technical components:

1. A streaming algorithm that produces a byte (or bit) stream from database data selected according to a set of criteria.
2. A one-way function-calculation routine for computing a one-way function, such as a hash function, of the byte stream produced from the selected database records. Computation-wise, such a routine can be analogous to conventional hash-code routines, such as MD2, MD5 and SHA, apart from the fact that it must operate on a byte stream instead of a document.
3. A public-key digital signature algorithm. A non-exhaustive list of suitable algorithms includes DSA, RSA and GOST.
4. A mechanism for securely storing and protecting the private key.
5. A mechanism for distributing the public keys and/or certificates certifying that a public key belongs to a certain user of the person or organization which performs the signing.
6. A mechanism for storing the digital signatures produced in such a way that they are available to all legitimate users.

The digital signature algorithm is preferably based on public key cryptography, whereby anyone having access to the public key is able to verify the signatures. In order to achieve a feasible technical implementation, the cryptographic signature algorithm, such as RSA or DSA, should be applied to the result of a one-way function, such as a hash function, computed over a document, instead of signing the whole document. A benefit of this technique is elimination of any overhead caused by processing the entire document. Instead of creating and storing an entire document, it suffices to transfer the result of the one-way function to a data processing apparatus which contains the private key used for signing it. For example, such a data processing apparatus may be a host computer, smart card or a high-security server environment for protecting the private key(s).

Naturally the general requirements of digital signatures should be met. Specifically, the signatures produced should ensure authenticity and be immune against forgery, alteration, re-use and repudiation.

As stated above, database records should be streamed to the signature algorithm, instead of generating a document to be signed. The streaming algorithm should meet the following requirements. First, if the database records that are in the scope of the data set to be signed are altered in any manner, or if any database records are added to or removed from the scope of the data set to be signed, the stream produced by the algorithm must be different from the stream corresponding to the original, unmodified records. Second, it must be possible to define the scope of the data set to be produced to the streaming algorithm such that all modifications, additions and deletions will cause the stream to differ from the original. Thus the way of defining the scope must be stable so that its semantics cannot be altered by changing some records in the database or by feeding some user input to the algorithm.

In order to ensure that any client application or human user perceives the signature as being bound to the actual database data instead of any concrete file, the streaming algorithm must also be reproducible. Reproducibility imposes the following requirements on the streaming algorithm. First, when applied repeatedly for the same data by different users it should always produce a document that consists exactly of the same bytes exactly in the same order. Second, the streaming algorithm should not be sensitive to the hardware/software platform (operating system proper, operating system versions, libraries, library versions, or the like). This requirement applies to the client computer(s), application server(s), tool server(s) and the database engine. Third, the streaming algorithm should not be sensitive to character encoding or localizations installed on the clients, application servers, tool servers or database engines.

Virtually any streaming algorithm which meets the above criteria can be used. CSV algorithms will be described as an illustrative but non-restricting example. The acronym 'CSV' stems from "comma-separated values", but as is well known, the published values can be separated by separators other than comma. For example, the tab character is frequently used as a separator. Rows are separated by a line feed and/or carriage return. Any character encoding, value demarcation and row demarcation must always remain the same, regardless of the hardware/software platform or other circumstances. The general steps of a CSV streaming algorithm are as follows:

1. Retrieve a set of full or partial records from the database, ordered by an attribute that produces a stable ordering.
2. If the database queries performed in step 1 do not produce a stable ordering, performing additional sorting which ensures a stable ordering of records.
3. Convert the records to CSV format by using some universal encoding, such as UTF8. A universally-accepted terminator, such as '\n', must be used to denote ends of lines. An end-of-file (EOF) must be returned only when there are no more records to be streamed.

For reasons of efficiency and scalability, the above steps can be per formed iteratively, in order to reduce memory consumption. Step 1 is the actual step where the content of the actual signed document is determined. This defines the scope of the signature.

Project head streaming defined step 1 of the CSV streaming algorithm with a step loading the data using the same project loader that is applied when a project is loaded to the experiment application. This involves a set of database queries that are not specified here. Here the additional sorting step 2 is needed, since project loader does not produce any predictable ordering for the data it fetches. An SQL code segment used for loading a project head efficiently is presented below. FOR each table, execute the following query: SELECT <columns in T> FROM <T> where <T>.owner_ project_id=?;

When a tagged version of a project is streamed, it involves fetching the tagged versions of data from tagged data tables of each table in the information model. The skeleton of the algorithm is the same as defined in the "General" chapter, but step 1 of the algorithm is defined below. The algorithm takes *tag_id* as its parameter.

1. Sort all domain tables (i.e. excluding system tables and temporary tables) into alphabetical order.
2. For each domain table <T> perform the following:

   a) Perform an inner join between the tag binder table *<T>_tb* and the tagged data table *<T>_tag,* where the join condition consists of *tag_id* which is set equal to the algorithm parameter *TAG_ID,* id which is set equal between the column in *<T>_tb* and *<T>* tag and *instance_version,* which is set equal between *<T>_tb* and *<T>_tag.* Order the records by *Id.* A SQL code segment for performing this operation is given below:

SELECT <columns in T>, <T>_tb.tag_id FROM <T>_tb, <T>_tag
WHERE <T>_tb.tag_id=? AND <T>_tb.id=<T>_tag.id AND
<T>_tb.instance_version=<T>_tag.instance_version;

b) Retrieve the tagged version of the working set of the tagged project; retrieve the working set tag binders and the corresponding tags and order them by *Id.* This is analogous to the operation presented above, except that *<T>* is set to *ws_tag_binder* and *working_set.*

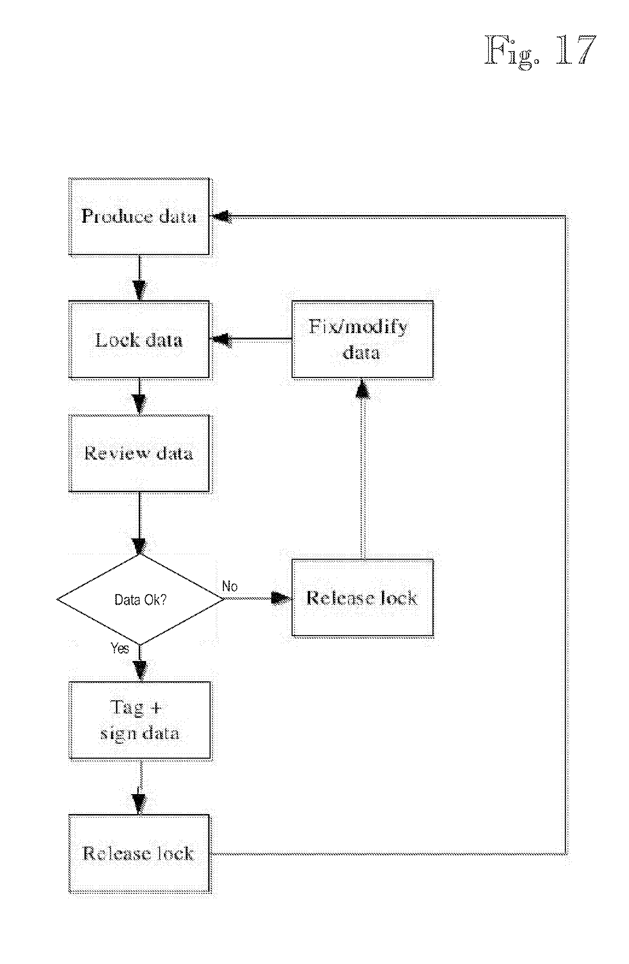

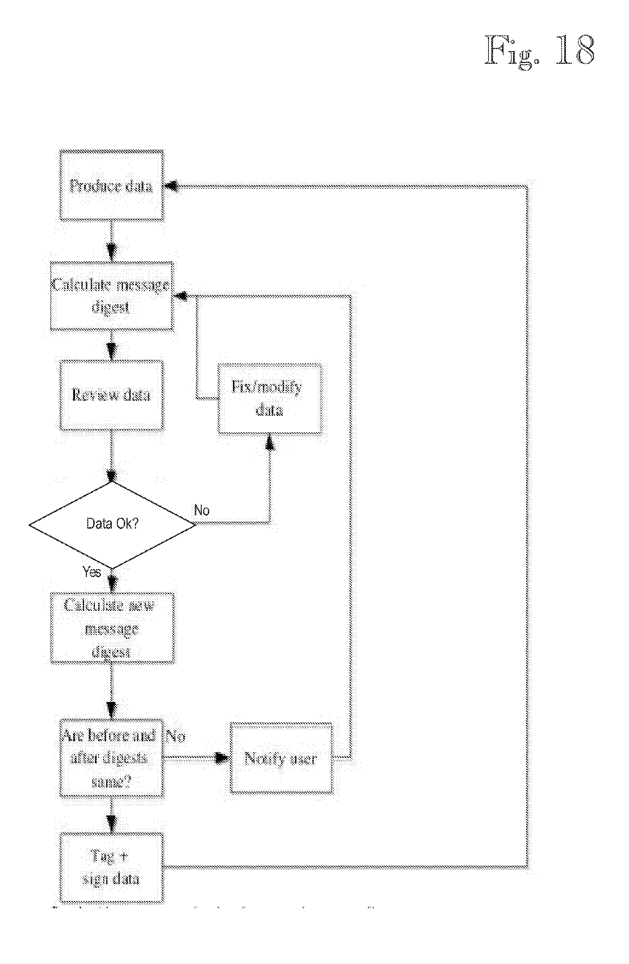

An acceptance procedure for accepting a produced data set will be described next, with reference to Figures 17 and 18.

Tagging is a natural place of acceptance procedure for produced data. After tagging, the tagged data does not change, whereby the

signature-application technique described above can be used. When inspecting data to be accepted, no modifications can be allowed because the changes made might not be reviewed at all. There two possible implementations of preventing changes during inspections will be described. One is based on locking the data, while the other is based on calculating a message digest from the data before inspection procedure and again after the process, and comparing the before- and after-digests. If the digests are the same, the data was not modified during the inspection and tagging may commence.

Figure 17 is flow chart illustrating an acceptance procedure based on locking of data. Locking the data can be implemented with relative ease if the relational database management system (RDBMS) provides such functionality. For example, Oracle Fine Grained Access Control (FGAC) allows preventing data manipulation based on a filter. In this case the filter can be as simple as *'owner_project_id <>* ?'. Here the question mark is a placeholder for the project id under the current acceptance procedure. This kind of a filter only allows updating those records that are part of any other project than the one being inspected. FGAC allows multiple filters at the same time, whereby multiple projects can be inspected at the same time. In the locking procedure shown in Figure 17, the data is first produced and then locked for inspection. If the data passes the review tests defined by application, the project data can be tagged for publication. After that, the lock is released so that users can continue producing new data. If the review tests fail and some modification is needed, the lock is released, data is fixed and the process starts over.

Figure 18 is flow chart illustrating an acceptance procedure based on a comparison of message digests. This procedure is similar to the lock-based procedure shown in Figure 17, but instead of preventing data modifications on that project, tagging is prohibited if the data was modified during the inspection. Unlike the lock-based procedure, the acceptance procedure shown in Figure 18 does not require any functionality from the underlying relational database management system.

It will be apparent to a person skilled in the art that, as the technology advances, the inventive concept can be implemented in various ways. The invention and its embodiments are not limited to the examples described above but may vary within the scope of the claims.

**Claims**

1.  A database management system for storing a plurality of biochemical data items which originate from one or more external data sources, the database management system comprising a database operable to store, for each biochemical data item:

    an objective identifier (OI) which comprises a set of attributes (143, 144) for characterizing measurable biochemical features of the biochemical data item; and
    a subjective identifier (SI) which comprises:

    an identity value (112);
    an identity type value (114, 122) which has an association (118) to the identity value; and
    a reference (128) to a data source, the reference being associated to the identity type value (114, 122).

2.  A database management system according to claim 1, further comprising an ETL logic (400, 500) for importing data items from at least one external data source into the database, wherein the ETL logic is operable to:

    test if both the subjective identity and objective identity of the data item to be imported matches those of an existing data item;

use the data item to be imported for updating the existing data item if the result of the test is positive; and to insert the data item to be imported as a new record if the result of the test is negative.

3.  A database management system according to claim 1, further comprising an ETL logic (600) for importing data items from at least one external data source into the database, wherein the ETL logic is operable to:

    test if either the subjective identity or objective identity of the data item to be imported matches those of an existing data item;
    use the data item to be imported for updating the existing data item if the result of the test is positive; and to insert the data item to be imported as a new record if the result of the test is negative.

4.  A database management system according to any one of the preceding claims, further comprising a name generation function (NG) for ensuring unique constraints in cases wherein two or more external data sources use identical values.

5.  A database management system according to any one of the preceding claims, further comprising a namespace mapping function for mapping one or more identities from one namespace to another.

6.  A database management system according to any one of the preceding claims, further comprising a logic (1002 - 1020) for identifying correspondence between entities originating from different data sources.

7.  A database management system according to any one of the preceding claims, further comprising a data version management logic, which comprises:

    means for maintaining a plurality of projects and for logging in a user to a project in response to a successful login;
    means for assigning one or more data sets to each of several projects such that data accessible to any project comprises one or more modifiable data sets assigned to the project in question and one or more tagged data sets assigned to other projects, wherein each tagged data set comprises a version number and is locked from modifications.

8.  A database management system according to claim 7, wherein the data version management logic further comprises means for maintaining a one or more subprojects under at least one parent project and for assigning data generated by the one or more subprojects to the parent project.

9.  A database management system according to claim 7 or 8, further comprising means for assigning a first table structure (1410) to modifiable data sets and a second table structure (1420) to tagged data sets.

10. A database management system according to any one of claims 7-9, further comprising means for producing tagged data sets by streaming and for assigning a digital signature to a streamed data set.

Fig. 1

100

145
144
143
142

140

**Entity**

| id |
| attribute1 |
| attribute2 |
| ... |
| identified_by |

119

115
114
113
112

110

**Identities**

| id |
| id_string |
| identity_type_id |
| entity_id |

118

124
123
122

120

**Identity_types**

| id |
| source_id |
| name |

128

133
132

130

**Source**

| id |
| name |

<attribute_1, attribute_2, ... , attribute n>

OI

| Objective identifier: |

145    143, 144

=  Entity.identified_by + columns specified by it

SI

| Subjective identifier: |

113    124    133

=  Identities.id_string + Identity_types.name + Source.name

Fig. 2

**250**

| source.name | identity_type.name | id_string | entity_id |
|---|---|---|---|
| EMBL | protein sequence ID | AAA18217.1 | 1 |
| EMBL | id | M29954 | 1 |
| PIR | id | A44945 | 1 |
| PIR | entry name | A44945 | 1 |
| TIGRFAMs | id | TIGR01870 | 1 |
| TIGRFAMs | entry name | cas_TM1810 | 1 |
| UniProt | protein name | 104 kDa microneme-rhoptry antigen | 1 |
| UniProt | entry name | 104K_THEPA | 1 |
| UniProt | accession | P15711 | 1 |

251

Identities

**210**

| id | id_string | identity_type_id | entity_id |
|---|---|---|---|
| 67118 | M29954 | 88 | 1 |
| 67119 | AAA18217.1 | 145 | 1 |
| 2745029 | TIGR01870 | 131 | 1 |
| 2745030 | cas_TM1810 | 57 | 1 |
| 1991356 | A44945 | 114 | 1 |
| 1991357 | A44945 | 50 | 1 |
| 2847709 | P15711 | 31 | 1 |
| 2847710 | 104K_THEPA | 58 | 1 |
| 2847711 | 104 kDa microneme-rhoptry antigen | 144 | 1 |

214   213   218

Identity_types

**220**

| id | name | source_id |
|---|---|---|
| 31 | accession | 94 |
| 57 | entry name | 90 |
| 58 | entry name | 94 |
| 50 | entry name | 66 |
| 88 | id | 25 |
| 114 | id | 66 |
| 131 | id | 90 |
| 144 | protein name | 94 |
| 145 | protein sequence ID | 25 |

224   223   228   232

Sources

**230**

| id | name |
|---|---|
| 25 | EMBL |
| 66 | PIR |
| 90 | TIGRFAMs |
| 94 | UniProt |

233   222

Fig. 3

Identities                                                                                    *310*

| id | id_string | identity_type_id | entity_id | source_id |
|----|-----------|------------------|-----------|-----------|
| 67118 | M29954 | 88 | 1 | |
| 67119 | AAA18217.1 | 145 | 1 | 94 |
| 2745029 | TIGR01870 | 131 | 1 | |
| 2745030 | cas_TM1810 | 57 | 1 | |
| 1991356 | A44945 | 114 | 1 | |
| 1991357 | A44945 | 50 | 1 | |
| 2847709 | P15711 | 31 | 1 | |
| 2847710 | 104K_THEPA | 58 | 1 | |

*317*

↑ 112     ↑ 113   ↑ 114              ↑ 115      ↑ 316

Sources                                    *230*

| id | name |
|----|------|
| 25 | EMBL |
| 66 | PIR |
| 90 | TIGRFAMs |
| 94 | UniProt |

*328*

↑ 132       *332*        *333*

Fig. 4

400

Start

402

Items to process? — No → End

Yes

Identify objective and subjective identities — 404

406

Objective identity exists? — No → | Insert as new record | 408

Yes

410

Subjective identity exists? — No → | Insert as new record | 412

Yes

| Update existing record | 414

500

Fig. 5

| Subjective identity / Objective identity | No match | Match |
|---|---|---|
| No match | Insert | Insert |
| Match | Insert | Update |

600

Fig. 6

| Subjective identity / Objective identity | No match | Match |
|---|---|---|
| No match | Insert | Update |
| Match | Update | Update |

Fig. 7

710 →

| name | molecular_weight | sequence | identified_by |
|------|------------------|----------|---------------|
| protein1 | 10 | ABC | SEQUENCE |

720 →

| name | molecular_weight | sequence | identified_by |
|------|------------------|----------|---------------|
| protein1 | 11 | ABC | SEQUENCE |

730 →

| name | molecular_weight | sequence | identified_by |
|------|------------------|----------|---------------|
| protein1_human | 11 | ABC | SEQUENCE |

740 →

| name | molecular_weight | sequence | identified_by |
|------|------------------|----------|---------------|
| protein1_mouse | 11 | ABC | SEQUENCE |

750 →

| name | molecular_weight | sequence | identified_by |
|------|------------------|----------|---------------|
| protein1_human | 11 | ABCD | SEQUENCE |

760 →

| name | molecular_weight | sequence | identified_by |
|------|------------------|----------|---------------|
| protein1_human/2 | 11 | ABCD | SEQUENCE |

Fig. 8

*812*

id_string='water'
datasource_id=ds1

*810*

ds1

compound
id_string='water'

NG

*814*

compound
id_string='water/1'

*822*

id_string='water'
datasource_id=ds2

*820*

ds2

compound
id_string='water'

NG

*824*

compound
id_string='water/2'

Fig. 9

900

```
select id_string
from identities i inner join
        identity_types idt on (i.identity_type_id = idt.id)
        inner join data_source ds on (idt.data_source_id = ds.id)
where ( i.object = ( ? )~910 )~916
        and idt.name = ( ? )~912
        and db.name = ( ? )~914
```

920

922

| id | name |
|---|---|
| 1 | 104K_THEPA |

| id_string |
|---|
| 104 kDa microneme-rhoptry antigen |

930

930

| id | name |
|---|---|
| 1 | 104K_THEPA |
| 2 | 108_LYCES |
| 3 | 10KD_VIGUN |
| 4 | 110K_PLAKN |
| 5 | 11S3_HELAN |
| 6 | 11SB_CUCMA |
| 7 | 120K_RICRI |
| 8 | 128U_DROME |
| 9 | 12AH_CLOS4 |
| 10 | 12KD_FRAAN |

| id_string | entity_id |
|---|---|
| 104 kDa microneme-rhoptry antigen | 1 |
| Protein 108 precursor | 2 |
| 10 kDa protein precursor | 3 |
| 110 kDa antigen | 4 |
| 11S globulin seed storage protein G3 precursor | 5 |
| 11S globulin beta subunit precursor | 6 |
| 120 kDa surface-exposed protein | 7 |
| GTP-binding protein 128UP | 8 |
| 12-alpha-hydroxysteroid dehydrogenase | 9 |
| Auxin-repressed 12.5 kDa protein | 10 |

934

| NAME | → | ID_STRING |
|---|---|---|
| 104K_THEPA | → | 104 kDa microneme-rhoptry antigen |
| 108_LYCES | → | Protein 108 precursor |
| 10KD_VIGUN | → | 10 kDa protein precursor |
| 110K_PLAKN | → | 110 kDa antigen |
| 11S3_HELAN | → | 11S globulin seed storage protein G3 precursor |
| 11SB_CUCMA | → | 11S globulin beta subunit precursor |
| 120K_RICRI | → | 120 kDa surface-exposed protein |
| 128U_DROME | → | GTP-binding protein 128UP |
| 12AH_CLOS4 | → | 12-alpha-hydroxysteroid dehydrogenase |
| 12KD_FRAAN | → | Auxin-repressed 12.5 kDa protein |

Fig. 10A

Start

Items to process? —1002 → No → End

Yes

Identify objective and subjective identities —1004

Objective identity exists? —1006 → No → Insert as new record —1008

Yes

Update existing record —1010

Fig. 10B

Start

Items to process? —1012 → No → End

Yes

Identify objective and subjective identities —1014

Subjective identity exists? —1016 → No → Insert as new record —1018

Yes

Update existing record —1020

Fig. 11

Category (or higher-level) contextual project

Contextual project

Subproject

Project Ontology — 1110

default — 1120

Populator — 1111

Research — 1112

p_uniprot — 1121
Produced tags: 1.0
2.0
1121D

p_kegg — 1122
1.7
1122D

p_bind — 1123
1.2

Allergy — 1113

Yeast — 1114

y1_analysis — 1124
2.6

1125W
Working set (union of children):
p_uniprot 2.0
p_uniprot 2.0-1.0  DEPRECATED
p_kegg 1.7
a1_analysis 2.4

1126W
Working set:
p_uniprot 1.0
p_kegg 1.7
a1_analysis 2.4

1141

1142

a1_allergy — 1125

a1_sample_production — 1126
2.3

a1_report — 1127
2.1

1127W
Working set:
p_uniprot 1.0
p_kegg 1.7
a1_analysis 2.4

1128W
Working set:
p_uniprot 2.0
p_kegg 1.7
a1_analysis 2.4

a1_analysis — 1128
2.4

1130  ...  1128D  1131  ...

1132  ...

Fig. 12

Accessible data

———◇ Consists of

Project data

Working set

Data record with owner_project_id

Tag

Live

Fixed

Head | Transferred

Deprecated | Internal | Deliverables

Fig. 13

Client

Versioning views

Active data of project

Defined by working set

Head tables (normal)

History tables

System tables

Tagged data+tag binder for each normal table

Fig. 14

Head tables

Primary key

1410

| id | 1411 |
| instance_version | 1412 |
| updated | 1413 |
| status | 1414 |
| modified_by | 1415 |

History tables, tagged tables

1420

| id | 1421 |
| instance_version | 1422 |
| updated | 1423 |
| status | 1424 |
| x_event | 1425 |
| x_retiretime | 1426 |
| modified_by | 1427 |

```
select id, c1, c2, c3, count(*) as cnt
from tag_table_x head join tag_table_x hist using(id, c1, c2, c3)
where hist.instance_version < head.instance_version
group by head.id, head.c1, head.c2, head.c3;
```
1440

Tag binders

1450

Primary key

| id | 1451 |
| instance_version | 1452 |
| tag_id | 1453 |

Tag tables

Primary key

1460

| id | 1461 |
| label | 1462 |

1500

Fig. 15

```
1502    select * from h_protein where project_id = current_project()
1506    union all
        select t_protein.* from t_protein where exists (
        select null from protein_tb
        where tag_id in get_working_set_tags()
1504    and protein_tb.id = t_protein.id
        and protein_tb.instance_version = t_protein.instance_version
        )
```

Fig. 16

```
                          Start

                                                    1602
        ┌────────────────────────────────────────────┐
        │  Create new record in tag table for the tag │
        └────────────────────────────────────────────┘
                            │                        1604
        ┌─────────────────────────────────────────────────┐
        │ Create new record in tag table for deprecated tag │
        └─────────────────────────────────────────────────┘
                            │                        1606
                  Tables to process in infomodel?  ──No──▶  End

                           Yes
                            │                        1608
                  Transferred tag of this table empty?  ──No──┐
                                                              │      1610
                           Yes                     1612        │  ┌──────────────┐
        ┌──────────────────────────────────────────┐         │  │   Error:      │
        │   Copy non-existing records to tagged table│         └─▶│ transferred tag│
        └──────────────────────────────────────────┘            │  not empty!   │
                            │                        1616        └──────────────┘
        ┌──────────────────────────────────────────┐
        │            Bind records to the tag         │
        └──────────────────────────────────────────┘
                            │                        1620
        ┌──────────────────────────────────────────┐
        │    Bind removed records to deprecated tag  │
        └──────────────────────────────────────────┘
```

1614

```
insert into tag_table_x (
        select * from head_table_x h
        where project_id = ? and not exists(
        select null from
        tag_table_x x where x.id = h.id
        and x.instance_version =
h.instance_version)
);
```

1618

```
insert into tb_x (
        select id, instance_version, ? as tag_id
        from head_table_x h
        where project_id = ?
)
```

1622

```
insert into tb_x (
        select *, ? as tag_id from (
                select id,
instance_version
                from head_table_x
                where tag_id = ?
        MINUS
                select id,
instance_version
                from head_table_x
                where tag_id = ?
        )
)
```

Fig. 17

Fig. 18